(19) Europäisches Patentamt
European Patent Office
Office européen des brevets

(11) EP 3 725 339 B1

(12) EUROPEAN PATENT SPECIFICATION

(45) Date of publication and mention of the grant of the patent:
**24.01.2024 Bulletin 2024/04**

(21) Application number: **18888507.3**

(22) Date of filing: **17.10.2018**

(51) International Patent Classification (IPC):
***A61L 31/10*** *(2006.01)* ***A61L 31/02*** *(2006.01)*
***A61L 31/14*** *(2006.01)* ***A61L 31/16*** *(2006.01)*
***A61L 27/04*** *(2006.01)* ***A61L 27/34*** *(2006.01)*
***A61L 27/54*** *(2006.01)* ***A61L 27/58*** *(2006.01)*

(52) Cooperative Patent Classification (CPC):
**A61L 27/042; A61L 27/34; A61L 27/54; A61L 27/58; A61L 31/022; A61L 31/10; A61L 31/148; A61L 31/16**

(86) International application number:
**PCT/CN2018/110575**

(87) International publication number:
**WO 2019/114406 (20.06.2019 Gazette 2019/25)**

# (54) ABSORBABLE IRON-BASED IMPLANTABLE DEVICE

ABSORBIERBARE IMPLANTIERBARE VORRICHTUNG AUF EISENBASIS

DISPOSITIF IMPLANTABLE À BASE DE FER RÉSORBABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2017 CN 201711346297**

(43) Date of publication of application:
**21.10.2020 Bulletin 2020/43**

(73) Proprietor: **Biotyx Medical (Shenzhen) Co., Ltd.**
**Shenzhen, Guangdong 518000 (CN)**

(72) Inventor: **QI, Haiping**
**Shenzhen, Guangdong 518057 (CN)**

(74) Representative: **Prinz & Partner mbB**
**Patent- und Rechtsanwälte**
**Rundfunkplatz 2**
**80335 München (DE)**

(56) References cited:
**EP-A1- 3 064 232**
**WO-A1-2018/145528**
**WO-A2-2007/111811**
**CN-A- 1 455 687**
**CN-A- 1 665 554**
**CN-A- 102 068 332**
**CN-A- 104 491 935**
**CN-A- 106 581 784**
**US-A1- 2004 037 886**
**US-A1- 2007 260 054**
**US-A1- 2009 202 610**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The embodiments relate to the field of interventional medical devices, in particular to an absorbable iron-based implantable device.

At present, the substrate material of the absorbable implantable medical device is mainly selected from polymers, magnesium-based materials, zinc alloys, iron-based materials, and the like. Polylactic acid is most widely used in polymer absorbable implantable medical devices, which has the advantages of complete degradation and absorption, the degradation products being carbon dioxide and water, and has the disadvantage of insufficient mechanical properties. Compared to metal-based devices, to meet the same mechanical properties, polymer-based devices need to be larger in size than metal-based devices, which limit the application of polymer-based devices. The magnesium-based material and the iron-based material have the advantages of easy to process and shape and high in mechanical strength, but due to the fact that the corrosion speed of the magnesium-based material in a human body is too fast, the mechanical property at the earlier stage of implantation can be met only by increasing the size of a magnesium-based device, which limits the application of the magnesium-based device as well.

The iron-based material exhibits a good biocompatibility as a substrate material of an implantable medical device, and the corrosion speed of the iron-based material is slower than that of a magnesium-based material, thus the iron-based device has a smaller size than both a polymer-based device and a magnesium-based device on the premise that the same mechanical properties at the earlier stage of implantation are met. However, the corrosion of iron-based materials in vivo can produce insoluble solid corrosion products such as $Fe(OH)_3$, FeOOH, FezOs, $Fe_3O_4$, $Fe_3(PO_4)_2$, and the like. The solid corrosion products are low in solubility, difficult to dissolve in body fluid, mostly exist in form of a loose precipitate and are difficult to be absorbed and metabolized by tissues, so that the absorption period of the implantable medical device is too long to cause long-term retention in the tissues, although the solid corrosion products can be absorbed and metabolized by the flow of intercellular fluid and engulfment of macrophages, but it would take a long time, resulting in a greatly prolonged absorption period of the implantable medical device.

US 2009/202610 A1 discloses a medical implant comprising a bioerodible metal portion and a coating overlying the bioerodible metal portion, wherein the coating comprises a therapeutic agent and a polysaccharide matrix which is reversibly cross-linked with polyvalent metal cations. After implantation into a body, the therapeutic agent is released and the bioerodible metal portion erodes under formation of polyvalent metal cations. The released polyvalent metal cations are then bound by the polysaccharide matrix, resulting in a re-cross-linking reaction of the polysaccharide matrix.

US 2004/037886 A1 shows a coating composition comprising at least one therapeutic agent dispersed in modified, biologically active binders, e.g. heparin, which can be hydrophobically modified. Optionally, the coating composition further includes a cap coating applied over the therapeutic agent and the binder. The coating can be applied on a medical implant, e.g. a stent.

EP 3 064 232 discloses an absorbable iron-based alloy stent, comprising an iron-based alloy substrate and a degradable polyester in contact with the surface of the substrate. The degradable polyester can be mixed with an active agent.

Therefore, how to reduce the insoluble solid corrosion products that the absorbable iron-based implantable device may produce is an urgent technical problem to be solved in the field.

For this reason, it is necessary to provide an absorbable iron-based implantable device capable of reducing insoluble solid corrosion products.

The object of the invention is solved by an absorbable iron-based implantable device according to claim 1.

The absorbable iron-based implantable device includes an iron-based substrate and a modified high polysaccharide attached to the iron-based substrate, where the modified high polysaccharide is a hydrophobically modified high polysaccharide as defined in claim 1.

In one embodiment, the modified high polysaccharide is made of a high polysaccharide grafted by a hydrophobic group.

In one embodiment, the hydrophobic group is a hydrocarbyl group having 8-24 carbon atoms; alternatively,

> the hydrophobic group is a hydrocarbyl group containing at least one group of aryl, hydroxyl, carboxyl, an ester group, an isocyanate group, an ether group, amine group and acyl or a hydrocarbyl group having a double bond; alternatively,
>
> the hydrophobic group is at least one selected from polyoxypropenyl, a long chain perfluoroalkyl and a polysiloxane group.

In one embodiment, the hydrophobic group is grafted to the high polysaccharide by reaction with hydroxyl, carboxyl, or amido of the high polysaccharide.

In one embodiment, the solubility of the modified high polysaccharide in water at 37 °C is less than or equal to 0.01 g, and the solubility of the modified high polysaccharide in an organic solvent is greater than or equal to 0.1 g.

In one embodiment, the degradation period of the modified high polysaccharide in vivo is more than 3 months.

In one embodiment, the degradation product of the modified high polysaccharide in vivo reacts with the iron-based substrate to produce a polysaccharide-iron complex which has a solubility of not less than 1 g in a physiological solution.

According to the claimed invention, the high polysaccharide is at least one selected from glucan, chitosan, plant polysaccharides, starch, dextrin, cellulose, glycogen, chitin, tunicin, inulin, agar, gum arabic, hyaluronic acid, gellan gum, curdlan, xanthan gum, pectin, konjac glucomannan, gum arabic, lichenin, alginate, spirulina polysaccharide, chondroitin sulfate, dermatan sulfate, keratan sulfate, heparin and heparan sulfate.

In one embodiment, the plant polysaccharide is ginseng polysaccharide, astragalus polysaccharide, angelica polysaccharide, lycium barbarum polysaccharide or capsicum polysaccharide.

In one embodiment, the modified high polysaccharide is located on the surface of the iron-based substrate or filled inside of the iron-based substrate.

In one embodiment, the absorbable iron-based implantable device further includes a degradable polymer layer disposed on the iron-based substrate, which is made of at least one material selected from a degradable polyester and a degradable polyanhydride, where the degradable polyester is any one selected from a group of polylactic acid, polyglycolic acid, poly(lactic-co-glycolic acid), polycaprolactone, polyhydroxyalkanoate, polyacrylate, polysuccinate, poly(β-hydroxybutyrate) and polyethylene adipate; or the degradable polyester is a physical blend of at least two selected from a group of polylactic acid, polyglycolic acid, polysuccinate, poly(β-hydroxybutyrate), polycaprolactone, polyethylene adipate, poly(lactic-co-glycolic acid) and polyhydroxybutyrate-valerate copolymer; or the degradable polyester is any one copolymer formed by copolymerization of at least two selected from monomers forming polylactic acid, polyglycolic acid, polysuccinate, poly(β-hydroxybutyrate), polycaprolactone, polyethylene adipate, poly(lactic-co-glycolic acid) and polyhydroxybutyrate-valerate copolymer; or the degradable polyanhydride is at least one selected from a group of poly(1,3-bis(p-carboxyphenoxy)propane-sebacic acid), poly(erucic acid dimer-sebacic acid) and poly(fumaric acid-sebacic acid); or the degradable polyanhydride is any one copolymer formed by copolymerization of at least two selected from monomers forming poly(1,3-bis(p-carboxyphenoxy)propane-sebacic acid), poly(erucic acid dimer-sebacic acid) and poly(fumaric acid-sebacic acid);

alternatively, the material of the degradable polymer layer is a copolymer formed by copolymerization of at least two monomers forming the degradable polyester and the degradable polyanhydride.

In one embodiment, the degradable polymeric layer further includes an active drug which is at least one selected from a group of antiangiogenic drugs, antiplatelet drugs, antithrombotic drugs, anti-inflammatory drugs, and anti-sensitizing drugs.

In one embodiment, the antiangiogenic drug is at least one selected from paclitaxel and rapamycin; the antiplatelet drug is cilostazol; the antithrombotic drug is heparin; the anti-inflammatory drug is dexamethasone; and the anti-sensitizing drug is at least one selected from calcium gluconate, chlorphenamine, and cortisone.

The mentioned-above absorbable iron-based implanted device includes an iron-based substrate and a hydrophobically modified high polysaccharide attached to the iron-based substrate, the solubility of the modified high polysaccharide in an organic solvent is increased when hydrophobically modified, this achieves a great film-forming property when a coating method such as spray coating or dip coating is employed for the preparation of a coating on the iron-base substrate, thus allowing an increased amount of the modified high polysaccharide to be attached to the iron-base substrate. Therefore, after the absorbable iron-based implantable device is implanted into a body, the degradation of the modified high polysaccharide generates more degradation products which react with the corrosion products of the iron-based substrate to produce a water-soluble polysaccharide-iron complex, thus reducing the generation of insoluble solid corrosion products.

The foregoing objects, features and advantages of the embodiments will become more apparent from the following detailed description.

Unless defined otherwise, all technical and scientific terms used herein have the same meaning as commonly understood by one of ordinary skill in the art to which the present disclosure belongs. The terms used herein in the description are for the purpose of describing particular embodiments only and not intended to be limiting.

When an absorbable iron-based implantable device is implanted into a body, the iron-based substrate is gradually corroded in a physiological solution to generate primary corrosion products $Fe^{2+}$ or $Fe^{3+}$, which then rapidly react with $OH^-$ to generate insoluble solid corrosion products such as $Fe(OH)_2$, $Fe(OH)_3$ and the like, and further generate insoluble products such as FeOOH, $Fe_2O_3$, $Fe_3O_4$ and the like; the reaction equations are shown in the following equations (1) to (3). These insoluble substances are loose in texture and may expand 3-8 times in volume relative to the iron-based substrate, and are insoluble in physiological solutions, resulting in a long period of metabolism and absorption by the tissue and a long-term retention in the tissue, thereby probably bringing some potential biological risks.

$$\mathrm{Fe} \xrightarrow{corrode} Fe^{2+}, Fe^{3+} \longrightarrow Fe(OH)_2, Fe(OH)_3 \qquad (1)$$

$$Fe(OH)_3 \xrightarrow{dehydration} FeOOH \xrightarrow{dehydration} Fe_2O_3 \qquad (2)$$

$$Fe(OH)_2 + Fe(OH)_3 \rightarrow Fe_3O_4 \qquad (3)$$

Accordingly, provided is an absorbable iron-based implantable device capable of reducing insoluble solid corrosion products.

According to the claimed invention, the absorbable iron-based implantable device includes an iron-based substrate and a modified high polysaccharide attached to the iron-based substrate, the modified high polysaccharide being a hydrophobically modified high polysaccharide as defined in claim 1. Hydrophobically modification refers to the improved hydrophobicity.

The iron-based substrate may be a pure iron substrate or an iron-based alloy substrate. For example, when the iron-based substrate is an iron-based alloy substrate, the iron-based alloy is an iron-based alloy having a carbon content of not more than 2.11 wt.%.

The modified high polysaccharide is formed by grafting a hydrophobic group to the high polysaccharide. The high polysaccharide may be a high homopolysaccharide of the same monosaccharide or may be a high heteropolysaccharide of different monosaccharides.

According to the invention, a high polysaccharide is at least one selected from glucan, chitosan, plant polysaccharide, starch, dextrin, cellulose, glycogen, chitin, tunicin, synanthrin, agar, Arabic gum, hyaluronic acid, gellan gum, curdlan, xanthan gum, pectin, konjac glucomannan, Arabic gum, seaweed lichen polysaccharide, alginate, spirulina polysaccharide, chondroitin sulfate, dermatan sulfate, keratan sulfate, heparin and heparan sulfate.

The plant polysaccharide is ginseng polysaccharide, astragalus polysaccharide, angelica polysaccharide, lycium barbarum polysaccharide or capsicum polysaccharide.

The high polysaccharide can be hydrolyzed in a physiological environment and converted into an oligosaccharide or a monosaccharide due to breakage of the glycosidic bond therein, and finally completely be hydrolyzed to obtain a monosaccharide with good biocompatibility and absorbability.

The hydrolysis product of the high polysaccharide can react with corrosion products of iron, such as iron hydroxide ($Fe(OH)_3$), iron oxyhydroxide (FeOOH) and the like to produce a water-soluble polysaccharide-iron complex. The core of the polysaccharide-iron complex is polymeric iron oxyhydroxide (or iron hydroxide), which is similar to ferritin. The polysaccharide-iron complex is free of iron ion and easily absorbed with little toxic and side effect, which can promote the hematopoietic function of an organism, thus effectively treating iron deficiency anemia.

Since the high polysaccharide itself is poorly soluble in water and organic solvents, it is difficult to attach the high polysaccharide to the iron-based substrate or the amount of high polysaccharide that the iron-based substrate can carry is small. The hydrophobicity of the high polysaccharide is improved by grafting hydrophobic groups, so that the solubility of the modified high polysaccharide in an organic solvent is improved, thereby facilitating the improvement of the carrying capacity of the high polysaccharide.

For example, the hydrophobic group is a hydrocarbyl group having 8-24 carbon atoms. Here, the C8-C24 hydrocarbyl group refers to a straight or branched hydrocarbyl group having 8-24 carbon atoms. The straight hydrocarbyl group may be straight alkyl or straight hydrocarbyl containing unsaturated bonds. The branched hydrocarbon group may be a branched alkyl or a branched hydrocarbyl containing unsaturated bonds.

For example, the hydrophobic group is a hydrocarbyl group containing at least one of aryl, hydroxyl, carboxyl, an ester group, an isocyanate group, an ether group, amine group and acyl or a hydrocarbon group containing a double bond.

The hydrophobic group containing at least one of aryl, hydroxyl, carboxyl, an ester group, an ether group, amine group and acyl refers to a straight or branched hydrocarbyl group containing at least one group of aryl, carboxyl, ester, ether, amine and amide. The hydrocarbyl group containing a double bond refers to a straight or branched hydrocarbyl group containing a double bond.

Alternatively, the hydrophobic group is at least one selected from polyoxypropenyl, a long chain perfluoroalkyl and a apolysiloxane group.

The modified high polysaccharide can be prepared from the high polysaccharide by reaction such as acylation, etherification, esterification, alkylation, silylation, graft copolymerization and the like.

The high polysaccharide is subjected to an acylation with an acylating agent, where aliphatic or aromatic acyl groups with different molecular weights are introduced into the high polysaccharide to form acylated high polysaccharides. The solubility of acylated high polysaccharides in organic solvents can be greatly improved compared to high polysaccharides.

The modified high polysaccharide can be prepared by the acylation reaction. For example, chitosan was suspended in dimethylformamide and heated to 130 °C, then was acylated by adding excess phthalic anhydride to obtain phthaloyl chitosan which is absorbable in dimethyl sulfoxide, the reaction equation being as follows:

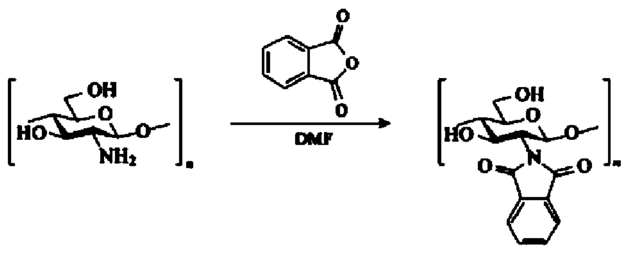


By etherification, hydroxyl of a high polysaccharide reacts with an alkylating agent (etherifying agent) to give a polysaccharide ether, i.e., a modified high polysaccharide. For example, etherification of cellulose by the effect of chloroethane (etherifying agent) can form ethyl cellulose ether. Ethyl cellulose ether is soluble in most organic solvents such as methanol, ethanol, benzene, toluene, xylene and the like.

In the case of acid catalysis, hydroxyl of a high polysaccharide is esterified with acid, acid anhydride, acyl halide and the like to obtain a polysaccharide ester, namely a modified high polysaccharide. For example, in the case of acid catalysis, cellulose is esterified with acetic anhydride to produce glucan oleate. Glucan oleate are soluble in organic solvents such as acetone, dimethylformamide and the like.

The high polysaccharide undergoes an alkylation reaction with an alkyl halide to produce an alkylated high polysaccharide. For example, benzaldehyde reacts with chitosan to form Schiff base followed by reduction with sodium borohydride to obtain benzyl chitosan.

By silylation reaction, the high polysaccharide reacts with silane to produce a silylated high polysaccharide. For example, chitin undergoes a silylation reaction with trimethylsilane to obtain trimethylsilyl chitin, the reaction equation being as follows:

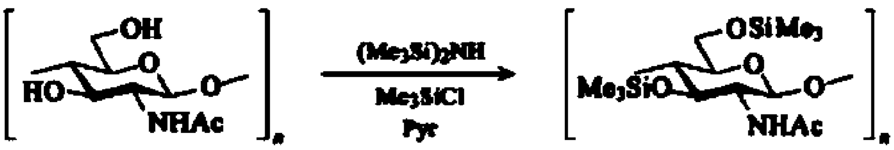


By graft copolymerization, a polysaccharide grafted copolymer can be prepared by the effect of initiators such as ammonium ceric nitrate, N,N-carbonyldiimidazole and the like. For example, a polycaprolactone-glucan copolymer which is soluble in organic solvents is prepared by using N,N-carbonyldiimidazole as an initiator.

By grafting the hydrophobic group to the high polysaccharide, it is favorable to improve the hydrophobicity of the high polysaccharide, thereby improving the solubility of the high polysaccharide in organic solvents.

The absorbable iron-based implantable device is used for implanting into a lesion site of a body to cure the lesion site, therefore, in order to ensure mechanical properties at the earlier stage, the absorbable iron-based implantable device cannot be corroded prematurely after implantation in the body, and the structure generally needs to be kept basically complete within 3 months after implantation. Moreover, the corrosion speed of the iron-based material itself is slow and the corrosion period is typically more than one year. In order to ensure that the high polysaccharide cannot be rapidly dissolved and diffused after implantation into a human body, the above hydrophobic group is grafted to the high polysaccharide, so that the solubility of the high polysaccharide in water is reduced, in order that the degradation period of the hydrophobically modified high polysaccharide in the human body is as consistent with the corrosion period of the iron-based substrate as possible, and the effect that insoluble solid corrosion products can be well reduced during the corrosion period of the iron-based substrate can be achieved as well as possible.

An appropriate high polysaccharide can be selected according to the corrosion period of the iron-based substrate, so that the high polysaccharide is matched with the iron-based substrate, and solid corrosion products of iron are not or less generated in the degradation process, thereby on one hand promoting metabolism and absorption of the absorbable iron-based implantable device, and on the other hand accelerating corrosion of the iron-based substrate.

For example, the solubility of the modified high polysaccharide in water at 37 °C is less than or equal to 0.01 g, and the solubility of the modified high polysaccharide in an organic solvent is greater than or equal to 0.1 g, in this way, the modified high polysaccharide has a longer degradation period in vivo and a great film formation, so that the iron-based substrate has a greater capacity for carrying the modified high polysaccharide.

For example, by selecting a suitable modified high polysaccharide, the modified high polysaccharide reacts with the iron-based substrate in a physiological solution to produce a polysaccharide-iron complex having a solubility (calculated as iron) of not less than 1 g in the physiological solution. The modified high polysaccharide is, for example,

a modified glucan, a modified plant polysaccharide.

For example, the molecular weight of the high polysaccharide in the modified high polysaccharide is 10,000-2,000,000. The degradation period of the high polysaccharide can be regulated by the molecular weight, the larger the molecular weight is, the longer the degradation period will be. For example, the high polysaccharide has a molecular weight of from 200,000 to 1,000,000 and a degradation period of from 3 months to 2 years to maximally match the corrosion period of the iron-based substrate.

The modified high polysaccharide may be attached to the surface of the iron-based substrate by means of a coating. For example, the modified high polysaccharide coating completely or partially covers the surface of the iron-based substrate. The surface of an iron-based substrate refers to the outer surface, inner surface and side of the iron-based substrate. The method of forming a coating on the surface of the iron-based substrate may be spray coating, spin coating, brush coating, electrospinning, etc.

In another embodiment, a corresponding groove or through-hole can also be provided inside of the iron-based substrate to fill the modified high polysaccharide into the groove or through-hole, so that the modified high polysaccharide is filled inside of the iron-based substrate.

In another embodiment, the absorbable iron-based implantable device further includes a degradable polymer layer disposed on the iron-based substrate. The degradable polymer layer may partially or completely cover the modified high polysaccharide coating, or the degradable polymer layer doesn't cover the modified high polysaccharide coating at all.

The degradable polymer layer is provided so that at the later stage of implantation when curing of the lesion site is completed, the degradable polymer layer is degraded to generate an acidic environment to accelerate the corrosion of the iron-based substrate, thereby reducing the probability of long-term adverse reactions.

The material of the degradable polymer layer is a degradable polymer. The degradable polymer is at least one selected from the degradable polyester and the degradable polyanhydride.

The degradable polyester is any one selected from polylactic acid, polyglycolic acid, poly(lactic-co-glycolic acid), polycaprolactone, polyhydroxyalkanoate, polyacrylate, polysuccinate, poly(β-hydroxybutyrate) and polyethylene adipate.

Alternatively, the degradable polyester is a physical blend of at least two selected from a group of polylactic acid, polyglycolic acid, polysuccinate, poly(β-hydroxybutyrate), polycaprolactone, polyethylene adipate, poly(lactic-co-glycolic acid), and polyhydroxybutyrate-valerate copolymer.

Alternatively, the degradable polyester is any one copolymer formed by copolymerization of at least two selected from monomers forming polylactic acid, polyglycolic acid, polysuccinate, poly(β-hydroxybutyrate), polycaprolactone, polyethylene adipate, poly(lactic-co-glycolic acid) and polyhydroxybutyrate-valerate copolymer.

The degradable polyanhydride is at least one selected from poly(1,3-bis (p-carboxyphenoxy) propane-sebacic acid), poly(erucic acid dimer-sebacic acid), and poly(fumaric acid-sebacic acid). Alternatively, the degradable anhydride is any one copolymer formed by copolymerization of at least two selected from monomers forming poly(1,3-bis(p-carboxyphenoxy)propane-sebacic acid), poly(erucic acid dimer-sebacic acid), and poly(fumaric acid-sebacic acid).

Alternatively, in a further embodiment, the material of the degradable polymer layer is a copolymer formed by copolymerization of at least two monomers forming the degradable polyester and the degradable polyanhydride described above.

The degradable polyester and the degradable polyanhydride described above show good biocompatibility with a human body, and acid substances can be generated after degradation thereof so as to accelerate the corrosion of the iron-based substrate.

For example, the degradable polymer layer further includes an active drug. The active drug is dispersed in the degradable polymer. The active drug is at least one selected from the group of antiangiogenic drugs, antiplatelet drugs, antithrombotic drugs, anti-inflammatory drugs, and anti-sensitizing drugs.

The antiangiogenic drug is at least one selected from taxol, rapamycin and rapamycin derivatives. The antiplatelet drug is cilostazol. The antithrombotic drug is heparin. The anti-inflammatory drug is dexamethasone. The anti-sensitizing drug is at least one selected from calcium gluconate, chlorpheniramine and cortisone.

The above-described absorbable iron-based implantable device includes an iron-based substrate and a hydrophobically modified high polysaccharide attached to the iron-based substrate, the solubility of the modified high polysaccharide in an organic solvent is increased when hydrophobically modified, this allows a great film formation on the iron-base substrate when a coating method such as spray coating or dip coating is employed for the preparation of a coating, thus allowing an increased amount of the modified high polysaccharide to be attached to the iron-base substrate. Therefore, when the absorbable iron-based implantable device is implanted into a body, the degradation of the modified high polysaccharide produces an increased amount of degradation product that reacts with the corrosion product of the iron-based substrate to produce a water-soluble polysaccharide-iron complex, thus reducing the production of an insoluble solid corrosion product.

Meanwhile, the above mentioned absorbable iron-based implantable device solves the problems that the polysaccharide shows a poor carrying capacity and quickly dissolves and diffuses in the body due to its poor film formation,

and can play a role mostly during the corrosion period of the iron-based substrate, namely play a long lasting effect.

The above-described absorbable iron-based implantable device may be a vascular stent, an orthopedic implant, a gynecological implant, an andrological implant, a respiratory implant or an orthopedic implant.

The following embodiments provide further examples and implementations.

The testing method adopted in the following embodiments is as follows:

First: The method for detecting the solubility of the modified high polysaccharide

A certain mass of modified high polysaccharide and 100 g solvent (water or organic solvent) were weighed. The modified high polysaccharide was gradually added into the solvent for multiple times at 25 °C, then stirred for completely dissolution until the modified high polysaccharide was just saturated (if solid precipitation occurred, then filtration and drying were performed to obtain the undissolved modified high polysaccharide). The mass of the undissolved modified high polysaccharide was weighed, and the mass of dissolved modified high polysaccharide was calculated as the solubility of the modified high polysaccharide at this temperature. In the actual test process, the amount of solute and solvent can be scaled down based on the actual situation.

Second: The concentration of the polysaccharide-iron complex is detected by the following method.

The absorbable iron-based implantable device was soaked in a phosphate buffer solution (PBS) with a pH value ranging from 7.4 ± 0.05, enabling the ratio of the volume of the PBS to the volume enclosed by the absorbable iron-based implantable device to be 5-10:1, where the device was required to be completely soaked in PBS, if the device was smaller while the soaking container was larger, the number of the devices can be increased. The physiological solution soaked with the absorbable iron-based implantable device was placed in a thermostatic water bath environment at 37 ± 1 °C and shaken at a rate of 40 to 80 rpm. At predetermined observation time points, such as 1 month, 3 months, 4 months, etc..., an aqueous film with aperture of 0.22 μm was used to remove the insoluble substance in the physiological solution by filtration, then the mass concentration of iron in the filtrate was measured by atomic absorption spectrometry (AAS), which is less than or equal to the solubility. At the same time, the corroded absorbable iron-based implantable device was placed in another PBS solution with the same volume for further corrosion until next observation time point, the testing process being as described above. If the elemental iron in the solution can be detected after corrosion in the exchanged solution for a period of time, it indicated that the polysaccharide-iron complex was gradually released. The solution was exchanged after 3 months, elemental iron in the exchanged solution can be detected after further corrosion for another period of time, it indicates that the degradation period of the modified high polysaccharide was more than 3 months.

Third: The measurement of the iron corrosion rate and the iron recovery rate

The iron corrosion rate detection method is as follows: the absorbable iron-based implantable device was implanted into an animal body, such as a rabbit iliac artery, where the mass of the iron-based substrate (referring to a bare stent that does not include a modified polysaccharide and a degradable polymer) was $M_0$. At a predetermined observation time point, animal was killed, and the absorbable iron-based implantable device implanted into the animal body as well as the tissues in which the device was located were intercepted, and soaked in a sodium hydroxide solution of 1 mol/L for tissue digestion, then the absorbable iron-based implantable device or fragments thereof were removed away from the solution and put into an organic solvent (such as acetonitrile) to remove the polymer at the surface by ultrasonic cleaning, and further put into a 3% tartaric acid solution to remove an iron corrosion product at the surface by ultrasonic cleaning. The remaining non-corroded absorbable iron-based implantable device or fragment thereof in the solution was removed, dried and weighed as a mass of $M_1$. The iron corrosion rate W is expressed as a percentage of the difference in the weight loss of the struts of the stent after corrosion and cleaning, based on the weight of the iron-based substrate, as shown in formula (4):

$$W = (M_0 - M_1)/M_0 \times 100\% \qquad \text{Formula (4)}$$

W --- Iron corrosion rate

$M_1$---Mass of remaining non-corroded iron-based stent substrate

$M_0$---Initial mass of iron-based stent substrate

The iron recovery rate test method is as follows: after mixing the above mentioned NaOH solution, the organic solution and the tartaric acid solution together, detect the concentration of the elemental iron in the solution using AAS, and calculating the mass of the iron in the solution as $M_2$. The iron recovery rate is calculated as shown in formula (5):

$$\text{Iron recovery} = (M_1 + M_2)/M_0 \times 100\% \qquad \text{Formula (5)}$$

Embodiment 1

Example 1 provided an absorbable iron-based stent including a stent substrate made of a pure iron material, and a glucan oleate coating prepared by spray coating to cover the surface of the stent substrate. The wall thickness of the stent substrate was 70 μm and the thickness of the glucan oleate coating was 50 μm. Glucan oleate has a molecular weight of 1,200,000. And glucan has a molecular weight of 1,000,000.

Glucan oleate was prepared by the acid catalysis method described above. Glucan oleate was insoluble in water and the solubility thereof in acetone was 7 g, as detected by the aforementioned testing method.

Five identical absorbable iron-based stents were made using the same material and method, and soaked together in a phosphate buffered saline (PBS) in a volume that is 5 times the volume of the stents. The PBS soaked with the absorbable iron-based stents was then placed in a thermostatic water bath environment at 37 °C and shaken at 60 rpm. The concentration of elemental iron in PBS was then detected by AAS after soaking for 1 month, 3 months, and 4 months, respectively. The detection results were as follows: the absorbable iron-based stent of Example 1 had an elemental iron concentration of 800 mg/L after soaking in PBS at 37 °C for 1 month. After soaking for 3 months, the concentration of elemental iron in PBS was 2000 mg/L. After soaking for 4 months, the concentration of elemental iron in PBS was 1200 mg/L. The results showed that the degradation period of glucan oleate was more than 3 months, the degradation product thereof reacted with the iron corrosion product to produce a water-soluble polysaccharide-iron complex.

The absorbable iron-based stent of Example 1 was implanted into a rabbit iliac artery, and the rabbit was killed after 12 months and the remaining absorbable iron-based stent was removed. The result obtained by testing was as follows: the iron corrosion rate was 40% and the iron recovery (including non-corroded iron, iron corrosion products, and iron corrosion products in the tissues surrounding the struts of the stent) was 70%, indicating that 30% iron was metabolized and absorbed.

Embodiment 2

Example 2 provided an absorbable iron-based stent including a stent substrate made of a pure iron material and a 2-hydroxyoctane-ginseng polysaccharide coating prepared by spray coating to cover the surface of the stent substrate. The wall thickness of the stent substrate was 70 μm and the thickness of the 2-hydroxyoctane-ginseng polysaccharide coating was 50 μm. The molecular weight of ginseng polysaccharide was 200,000.

2-hydroxyoctane-ginseng polysaccharide was prepared by addition reaction of 1,2-epoxyhexane and ginseng polysaccharide under the base catalysis. 2-hydroxyoctane-ginseng polysaccharide was insoluble in water and the solubility thereof in dichloromethane was 3 g, as detected by the above testing method.

Five identical absorbable iron-based stents were made using the same material and method, and the five absorbable iron-based stents were soaked together in phosphate buffered saline (PBS) in a volume that is 5 times the volume of the stents. The PBS soaked with the absorbable iron-based stents was then placed in a thermostatic water bath environment at 37 °C and shaken at 60 rpm. The concentration of elemental iron in PBS was then detected by AAS after shaking for 1 month, 3 months, and 4 months, respectively. The detection results were as follows: the absorbable iron-based stent of Example 2 had an elemental iron concentration of 700 mg/L after soaking in PBS at 37 °C for 1 month. After soaking for 3 months, the concentration of iron in PBS was 1800 mg/L. After soaking for 4 months, the concentration of iron in PBS was 800 mg/L. The results showed that the degradation period of 2-hydroxyoctane-ginseng polysaccharide was more than 3 months, and the degradation product thereof reacted with the iron corrosion product to produce a water-soluble polysaccharide-iron complex.

Embodiment 3

Example 3 provided an absorbable iron-based stent including a stent substrate made of nitriding iron material, an n-octane-glucan coating prepared by spray coating to cover the surface of the stent substrate, and a poly-DL-lactic acid coating on the surface of the n-octane-glucan coating. The wall thickness of the stent substrate was 60 μm, the thickness of the n-octane-glucan coating was 50 μm, and the thickness of the poly poly-DL-lactic acid coating was 4 μm. Glucan has a molecular weight of 1,000,000.

N-octane-glucan is prepared by the addition reaction of n-octyl chloride and glucan under the base catalyses. N-octane-glucan was insoluble in water and the solubility thereof in toluene was 5 g, as deteced by the above testing method.

Five identical absorbable iron-based stents were made using the same material and method, and soaked together in phosphate buffered saline (PBS) in a volume that is 5 times the volume of the stents. The PBS soaked with the absorbable iron-based stents was then placed in a thermostatic water bath environment at 37 °C and shaken at 60 rpm. The concentration of iron in the PBS was then detected by AAS after shaking for 1 month, 3 months, and 4 months, respectively. The detection results were as follows: the absorbable iron-based stent of Example 3 had an elemental iron concentration of 470 mg/L after soaking in PBS at 37 °C for 1 month. After soaking for 3 months, the concentration of elemental iron in PBS was 1100 mg/L. After soaking for 4 months, the concentration of elemental iron in PBS was 750 mg/L. The results showed that the degradation period of n-octane-glucan was more than 3 months, and the degradation products of n-octane-glucan reacted with the iron corrosion product to produce a water-soluble polysaccharide-iron complex.

Embodiment 4

Example 4 provided an absorbable iron-based stent including a stent substrate made of nitriding iron material, and a glucan hexadecyl cyanate coating prepared by spray coating to cover the surface of the stent substrate, and a poly-DL-lactic acid coating on the surface of the glucan hexadecyl cyanate coating. The wall thickness of the stent substrate was 60 μm, the thickness of the hexadecyl cyanate-glucan coating was 50 μm, and the thickness of the poly-DL-lactic acid coating was 4 μm. Glucan has a molecular weight of 800,000.

Glucan hexadecyl cyanate was prepared by the addition reaction of the isocyanate group. Glucan hexadecyl cyanate was insoluble in water and the solubility thereof in chloroform is 7 g, as detected by the above testing method.

Five identical absorbable iron-based stents were made using the same material and method, and soaked together in phosphate buffered saline (PBS) in a volume that is 5 times the volume of the stents. The PBS soaked with the absorbable iron-based stents was then placed in a thermostatic water bath environment at 37 °C and shaken at 60 rpm. The concentration of elemental iron in the PBS was then detected by AAS while shaking for 1 month, 3 months, and 4 months, respectively. The detection results were as follows: the absorbable iron-based stent of Example 4 had an elemental iron concentration of 540 mg/L after soaking in PBS at 37 °C for 1 month. After soaking for 3 months, the concentration of elemental iron in PBS was 1320 mg/L. After soaking for 4 months, the concentration of elemental iron in PBS was 890 mg/L. The results showed that the degradation period of glucan hexadecyl cyanate was more than 3 months, the degradation product thereof reacted with the iron corrosion product to produce a water-soluble polysaccharide-iron complex.

Embodiment 5

Example 5 provided an absorbable iron-based stent including a stent substrate made of nitriding iron material and a polyoxypropylene glyceryl ether-starch coating prepared by spray coating to cover the surface of the stent substrate. The wall thickness of the stent substrate was 70 μm and the thickness of the polyoxypropylene glycerol ether-starch coating was 50 μm. Starch had a molecular weight of 500,000.

Polyoxypropylene glycerol ether-starch was prepared by the addition reaction of a double bond. Polyoxypropylene glyceryl ether-starch was insoluble in water and the solubility thereof in acetone was 2 g, as detected by the above testing method.

Five identical absorbable iron-based stents were made using the same material and method, and soaked together in phosphate buffered saline (PBS) in a volume that is 5 times the volume of the stents. The PBS soaked with the absorbable iron-based stents was then placed in a thermostatic water bath environment at 37 °C and shaken at 60 rpm. The concentration of elemental iron in PBS was then detected by AAS while shaking for 1 month, 3 months, and 4 months, respectively. The detection results were as follows: the absorbable iron-based stent of Example 5 had an elemental iron concentration of 240 mg/L after soaking in PBS at 37 °C for 1 month. After soaking for 3 months, the concentration of elemental iron in PBS was 620 mg/L. After soaking for 4 months, the concentration of elemental iron in PBS was 390 mg/L. The results showed that the degradation period of polyoxypropylene glycerol ether-starch was more than 3 months, and the degradation product thereof reacted with the iron corrosion product to produce a water-soluble polysaccharide-iron complex.

Embodiment 6

Example 6 provides an absorbable iron-based stent including a stent substrate made of nitriding iron material, and a perfluoroalkylethyl acrylate-chitosan coating prepared by spray coating to cover the surface of the stent substrate.

The wall thickness of the stent substrate was 60 μm and the thickness of the perfluoroalkylethyl acrylate-chitosan was 60 μm. Chitosan had a molecular weight of 1,200,000.

Perfluoroalkylethyl acrylate-chitosan was prepared by the addition reaction of a double bond. Perfluoroalkylethyl acrylate-chitosan was insoluble in water and the solubility thereof in dichloromethane was 8 g, as detected by the above testing method.

Five identical absorbable iron-based stents were made using the same material and method, and soaked together in phosphate buffered saline (PBS) in a volume that is 5 times the volume of the stents. The PBS soaked with the absorbable iron-based stents was then placed in a thermostatic water bath environment at 37 °C and shaken at 60 rpm. The concentration of iron in PBS was then detected by AAS after shaking for 1 month, 3 months, and 4 months, respectively. The detection results were as follows: the absorbable iron-based stent of Example 6 had an elemental iron concentration of 190 mg/L after soaking in PBS at 37 °C for 1 month. After soaking for 3 months, the concentration of elemental iron in PBS was 570 mg/L. After soaking for 4 months, the concentration of elemental iron in PBS was 270 mg/L. The results showed that the degradation period of perfluoroalkyl ethyl acrylate-chitosan was more than 3 months, and the degradation product thereof reacted with the iron corrosion product to produce a water-soluble polysaccharide-iron complex.

Embodiment 7

Example 7 provided an absorbable iron-based stent including a stent substrate made of a pure iron material, a polycaprolactone-glucan copolymer coating prepared by spray coating to cover the surface of the stent substrate, and a poly-DL-lactic acid coating on the surface of the polycaprolactone-glucan copolymer coating. Rapamycin is contained in the poly-DL-lactic acid coating with a mass percentage of 50%. The wall thickness of the stent substrate was 70 μm, the thickness of the polycaprolactone-glucan copolymer coating was 50 μm, and the thickness of the poly-DL-lactic acid coating was 4 μm. Glucan had a molecular weight of 500,000.

The polycaprolactone-glucan copolymer was prepared by the above graft copolymerization method using N, N-carbonyldiimidazole as an initiator. The polycaprolactone-glucan copolymer was insoluble in water and the solubility thereof in acetone was 2 g, as detected by the above testing method.

Five identical absorbable iron-based stents were made using the same material and method, and soaked together in phosphate buffered saline (PBS) in a volume that is 5 times the volume of the stents. The PBS soaked with the absorbable iron-based stents was then placed in a thermostatic water bath environment at 37 °C and shaken at 60 rpm. The concentration of elemental iron in PBS was then detected by AAS after shaking for 1 month, 3 months, and 4 months, respectively. The detection results were as follows: the absorbable iron-based stent of Example 7 had an elemental iron concentration of 360 mg/L after soaking in PBS at 37 °C for 1 month After 3 months of immersion, the concentration of elemental iron in PBS was 950 mg/L. After soaking for 4 months, the concentration of elemental iron in PBS was 530 mg/L. The results showed that the degradation period of polycaprolactone-glucan copolymer was more than 3 months, and the degradation products thereof reacted with the iron corrosion product to produce a water-soluble polysaccharide-iron complex.

Embodiment 8

Example 8 provided an absorbable iron-based stent including a stent substrate made of nitriding iron material, and a trimethylsilyl chitin coating prepared by spray coating to cover the surface of the stent substrate, and a poly-DL-lactic acid coating on the surface of the trimethylsilyl chitin coating. Rapamycin was contained in the poly-DL-lactic acid coating with a mass percentage of 30%. The wall thickness of the stent substrate was 70 μm, the thickness of the trimethylsilyl chitin coating was 50 μm, and the thickness of the poly-DL-lactic acid coating was 10 μm. Chitin had a molecular weight of 200,000.

Trimethylsilyl chitin was prepared by the above silylation reaction. Trimethylsilyl chitin was insoluble in water and the solubility thereof in acetone was 12 g, as detected by the above testing method.

Five identical absorbable iron-based stents were made using the same material and method, and soaked together in phosphate buffered saline (PBS) in a volume that is 5 times the volume of the stents. The PBS soaked with the absorbable iron-based stents was then placed in a thermostatic water bath environment at 37 °C and shaken at 60 rpm. The concentration of iron in PBS was then detected by AAS after shaking for 1 month, 3 months, and 4 months, respectively. The detection results were as follows: the absorbable iron-based stent of Example 8 had an elemental iron concentration of 570 mg/L after soaking in PBS at 37 °C for 1 month. After soaking for 3 months, the concentration of iron in PBS was 1350 mg/L. After soaking for 4 months, the concentration of iron in PBS was 700 mg/L. The results showed that the degradation period of trimethylsilyl chitin was more than 3 months, the degradation product thereof reacted with the iron corrosion product to produce a water-soluble polysaccharide-iron complex.

Embodiment 9

Example 9 provided an absorbable iron-based stent including a stent substrate made of nitriding iron material and an N-phthaloyl chitosan coating prepared by spray coating to cover the surface of the stent substrate. The wall thickness of the stent substrate was 60 μm and the thickness of the N-phthaloyl chitosan coating was 50 μm. Chitosan had a molecular weight of 200,000.

N-phthaloyl chitosan was prepared by the above acylation method. N-Phthaloyl chitosan was insoluble in water and the solubility thereof in dimethyl sulfoxide was 1 g, as detected by the above testing method.

Five identical absorbable iron-based stents were made using the same materials and methods, and soaked together in phosphate buffered saline (PBS) in a volume that is 5 times the volume of the stents. The PBS soaked with the absorbable iron-based stents was then placed in a thermostatic water bath environment at 37 °C and shaken at 60 rpm. The concentration of iron in PBS was then detected by AAS while shaking for 1 month, 3 months, and 4 months, respectively. The detection results were as follows: the absorbable iron-based stent of Example 9 had an elemental iron concentration of 100 mg/L after soaking in PBS at 37 °C for 1 month. After soaking for 3 months, the concentration of iron in PBS was 320 mg/L. After soaking for 4 months, the concentration of iron in PBS was 240 mg/L. The results showed that the degradation period of N-phthaloyl chitosan was more than 3 months, the degradation product thereof reacted with the iron corrosion product to produce a water-soluble polysaccharide-iron complex.

Embodiment 10

Example 10 provided an absorbable iron-based stent including a stent substrate made of a pure iron material, a benzyl chitosan coating prepared by spray coating to cover the surface of the stent substrate, and a poly-DL-lactic acid coating on the surface of the benzyl chitosan coating. Rapamycin was contained in the poly-DL-lactic acid coating with a mass percentage of 50%. The wall thickness of the stent substrate was 60 μm, the thickness of the benzyl chitosan coating was 60 μm, and the thickness of the poly(racemic lactic acid) coating was 5 μm. Chitosan had a molecular weight of 300,000.

Benzyl chitosan was prepared by the alkylation reaction described above. Benzyl chitosan was insoluble in water and the solubility thereof in chloroform was 3 g, as detected by the above testing method.

Five identical absorbable iron-based stents were made using the same material and method, and soaked together in phosphate buffered saline (PBS) in a volume that is 5 times the volume of the stents. The PBS soaked with the absorbable stents was then placed in a thermostatic water bath environment at 37 °C and shaken at 60 rpm. The concentration of elemental iron in PBS was then detected by AAS after shaking for 1 month, 3 months, and 4 months, respectively. The detection results were as follows: the absorbable iron-based stent of Example 10 had an elemental iron concentration of 500 mg/L after soaking in PBS at 37 °C for 1 month. After soaking for 3 months, the concentration of elemental iron in PBS was 1200 mg/L. After soaking for 4 months, the concentration of elemental iron in PBS was 400 mg/L. The results showed that the degradation period of benzyl chitosan was more than 3 months, the degradation product thereof reacted with the iron corrosion product to produce a water-soluble polysaccharide-iron complex.

Comparative Example 1

Five identical absorbable nitriding iron-based stents were made then soaked together in phosphate buffered saline (PBS) with a volume that is 5 times the volume of the stents. The PBS soaked with the absorbable nitriding iron-based stents was then placed in a thermostatic water bath environment at 37 °C and shaken at 60 rpm. The concentration of elemental iron in PBS was then detected by AAS after shaking for 1 month, 3 months, and 4 months, respectively. The detection results were as follows: the absorbable nitriding iron-based stent of the Comparative Example 1 had an elemental iron concentration of 0 mg/L after soaking in PBS at 37 °C for 1 month. After soaking for 3 months, the concentration of iron in PBS was 0 mg/L. After soaking for 4 months, the concentration of iron in PBS was 0 mg/L.

The absorbable iron-based stents of Comparative Example 1 were implanted into iliac arteries of rabbits, the rabbits were sacrificed after 12 months, and the remaining absorbable iron-based stents were removed out. The result obtained by testing was as follows: the Iron corrosion rate was 20% and the iron recovery (including non-corroded iron, the iron corrosion products and iron corrosion products in the tissue surrounding the struts of the stent) was 95%, indicating that 5% of iron was metabolized and absorbed.

Compared with Comparative Example 1, the absorbable iron-based stents of Example 1-10 produced a water-soluble polysaccharide-iron complex with a solubility of greater than 1 g in a physiological environment due to the presence of the modified high polysaccharide, while Comparative Example 1 producing insoluble solid corrosion products. Therefore, the absorbable iron-based implantable device of Examples 1-10 reduced the generation of insoluble solid corrosion products, making the corrosion product of the iron-based substrate easy to be metabolized and absorbed.

By comparing Example 1 with Comparative Example 1, due to the presence of the modified high polysaccharide

in the stent of Example 1, after the absorbable iron-based stent of Example 1 and that of Comparative Example 1 being implanted into the rabbit iliac artery for 6 months, respectively, the data showed that Example 1 had a faster corrosion speed than Comparative Example 1, as well as a greater percentage of iron metabolism and absorption than that of Comparative Example 1.

The various technical features of the above-mentioned embodiments may be combined in any way, and in order to simplify the description, not all possible combinations of the technical features of the above-mentioned embodiments are described, however, as long as there is no conflict between these technical features, they should be considered to be within the scope of the description.

## Claims

1. An absorbable iron-based implantable device, comprising:

   an iron-based substrate, and
   a modified high polysaccharide attached to the iron-based substrate,
   wherein the high polysaccharide is at least one selected from glucan, chitosan, plant polysaccharides, starch, dextrin, cellulose, glycogen, chitin, tunicin, inulin, agar, gum arabic, hyaluronic acid, gellan gum, curdlan, xanthan gum, pectin, konjac glucomannan, gum arabic, lichenin, alginate, spirulina polysaccharide, chondroitin sulfate, dermatan sulfate, keratan sulfate, heparin and heparan sulfate, and
   wherein the modified high polysaccharide is a hydrophobically modified high polysaccharide.

2. The absorbable iron-based implantable device of claim 1, wherein the modified high polysaccharide is formed by grafting a hydrophobic group to a high polysaccharide.

3. The absorbable iron-based implantable device of claim 2, wherein the hydrophobic group is a hydrocarbyl group having 8-24 carbon atoms; or

   the hydrophobic group is a hydrocarbyl group containing at least one of aryl, hydroxyl, carboxyl, an ester group, an isocyanate group, an ether group, amine group and acyl or a hydrocarbyl group containing a double bond; or the hydrophobic group is at least one selected from polyoxypropenyl, a long chain perfluoroalkyl and a polysiloxane group.

4. The absorbable iron-based implantable device of claim 2, wherein the hydrophobic group reacts with hydroxyl, carboxyl, or the amino group of the high polysaccharide to graft to the high polysaccharide.

5. The absorbable iron-based implantable device of claim 1, wherein the solubility of the modified high polysaccharide in water at 37 °C is less than or equal to 0.01 g, and the solubility of the modified high polysaccharide in an organic solvent is greater than or equal to 0.1 g.

6. The absorbable iron-based implantable device of claim 1, wherein the degradation period of the modified high polysaccharide in vivo is greater than 3 months, and
   wherein determination of the degradation period includes: soaking the implantable device in phosphate buffered saline in a volume that is 5 times the volume of the implantable device and placing the implantable device in a thermostatic water bath environment at 37 °C shaken at 60 rpm for greater than 3 months.

7. The absorbable iron-based implantable device of claim 1, wherein a degradation product of the modified high polysaccharide in vivo reacts with the iron-based substrate to form a polysaccharide-iron complex having a solubility in a physiological solution of not less than 1 g.

8. The absorbable iron-based implantable device of claim 1, wherein the plant polysaccharide is ginseng polysaccharide, astragalus polysaccharide, angelica polysaccharide, lycium barbarum polysaccharide or capsicum polysaccharide.

9. The absorbable iron-based implantable device of claim 1, wherein the modified high polysaccharide is located on a surface of the iron-based substrate, or the modified high polysaccharide is filled inside of the iron-based substrate.

10. The absorbable iron-based implantable device of claim 1, wherein the absorbable iron-based implantable device

further comprises a degradable polymer layer disposed on the iron-based substrate, and

the degradable polymer layer is made of at least one material selected from a degradable polyester and a degradable polyanhydride,
wherein the degradable polyester is any one selected from the group consisting of polylactic acid, polyglycolic acid, poly(lactic-co-glycolic acid), polycaprolactone, polyhydroxyalkanoates, polyacrylates, polysuccinates, poly(β-hydroxybutyrate) and polyethylene adipate; or
the degradable polyester is a physical blend of at least two selected from the group consisting of polylactic acid, polyglycolic acid, polysuccinate, poly(β-hydroxybutyrate), polycaprolactone, polyethylene adipate, poly(lactic-co-glycolic acid) and polyhydroxybutyrate-valerate copolymer; or
the degradable polyester is any one copolymer formed by copolymerization of at least two selected from monomers forming polylactic acid, polyglycolic acid, polysuccinate, poly(β-hydroxybutyrate), polycaprolactone, polyethylene adipate, poly(lactic-co-glycolic acid) and polyhydroxybutyrate-valerate copolymer; or
the degradable polyanhydride is at least one selected from the group consisting of poly(1,3-bis(p-carboxyphenoxy) propane-sebacic acid), poly(erucic acid dimer-sebacic acid) and poly(fumaric acid-sebacic acid); or
the degradable polyanhydride is any one copolymer formed by copolymerization of at least two selected from monomers forming poly(1,3-bis(p-carboxyphenoxy) propane-sebacic acid), poly(erucic acid dimer-sebacic acid) and poly(fumaric acid-sebacic acid);
or, the material of the degradable polymer layer is a copolymer formed by copolymerization of at least two monomers forming the degradable polyester and the degradable polyanhydride.

**11.** The absorbable iron-based implantable device of claim 10, wherein the degradable polymeric layer further comprises an active drug which is at least one selected from the group consisting of antiangiogenic drugs, antiplatelet drugs, antithrombotic drugs, anti-inflammatory drugs, and anti-sensitizing drugs.

**12.** The absorbable iron-based implantable device of claim 11, wherein

the antiangiogenic drug is selected from at least one of paclitaxel and rapamycin;
the antiplatelet drug is cilostazol;
the antithrombotic drug is heparin;
the anti-inflammatory drug is dexamethasone; and
the anti-sensitizing drug is at least one selected from calcium gluconate, chlorphenamine, and cortisone.

## Patentansprüche

**1.** Implantierbare Vorrichtung auf der Basis von resorbierbarem Eisen, die Folgendes umfasst:

ein Substrat auf Eisenbasis und
ein modifiziertes hohes Polysaccharid, das an das Substrat auf Eisenbasis gebunden ist,
wobei das hohe Polysaccharid mindestens eines ist, das aus Glucan, Chitosan, pflanzlichen Polysacchariden, Stärke, Dextrin, Cellulose, Glykogen, Chitin, Tunicin, Inulin, Agar, Gummi arabicum, Hyaluronsäure, Gellangummi, Curdlan, Xanthangummi, Pektin, Konjak-Glucomannan, Gummi arabicum, Lichenin, Alginat, Spirulina-Polysaccharid, Chondroitinsulfat, Dermatansulfat, Keratansulfat, Heparin und Heparansulfat ausgewählt ist, und
wobei das modifizierte hohe Polysaccharid ein hydrophob modifiziertes hohes Polysaccharid ist.

**2.** Implantierbare Vorrichtung auf der Basis von resorbierbarem Eisen nach Anspruch 1, wobei das modifizierte hohe Polysaccharid durch Aufpfropfen einer hydrophoben Gruppe auf ein hohes Polysaccharid gebildet ist.

**3.** Implantierbare Vorrichtung auf der Basis von resorbierbarem Eisen nach Anspruch 2, wobei die hydrophobe Gruppe eine Hydrocarbylgruppe mit 8 bis 24 Kohlenstoffatomen ist oder

die hydrophobe Gruppe eine Hydrocarbylgruppe ist, die Aryl und/oder Hydroxyl und/oder Carboxyl und/oder eine Estergruppe und/oder eine Isocyanatgruppe und/oder eine Ethergruppe und/oder eine Amingruppe und Acyl oder eine eine Doppelbindung enthaltende Hydrocarbylgruppe enthält, oder
die hydrophobe Gruppe mindestens eine ist, die aus Polyoxypropenyl, einem langkettigen Perfluoralkyl und einer Polysiloxangruppe ausgewählt ist.

4. Implantierbare Vorrichtung auf der Basis von resorbierbarem Eisen nach Anspruch 2, wobei die hydrophobe Gruppe zum Aufpfropfen auf das hohe Polysaccharid mit Hydroxyl, Carboxyl oder der Aminogruppe des hohen Polysaccharids reagiert.

5. Implantierbare Vorrichtung auf der Basis von resorbierbarem Eisen nach Anspruch 1, wobei die Löslichkeit des modifizierten hohen Polysaccharids in Wasser bei 37°C kleiner oder gleich 0,01 g ist und die Löslichkeit des modifizierten hohen Polysaccharids in einem organischen Lösungsmittel größer oder gleich 0,1 g ist.

6. Implantierbare Vorrichtung auf der Basis von resorbierbarem Eisen nach Anspruch 1, wobei die Abbauzeit des modifizierten hohen Polysaccharids in vivo mehr als 3 Monate beträgt und
wobei die Bestimmung der Abbauzeit Folgendes umfasst: Tränken der implantierbaren Vorrichtung in phosphatgepufferter Kochsalzlösung in einem Volumen, das dem Fünffachen des Volumens der implantierbaren Vorrichtung entspricht, und Anordnen der implantierbaren Vorrichtung in einer thermostatischen Wasserbadumgebung bei 37°C unter Schütteln bei 60 U/min mehr als 3 Monate lang.

7. Implantierbare Vorrichtung auf der Basis von resorbierbarem Eisen nach Anspruch 1, wobei ein Abbauprodukt des modifizierten hohen Polysaccharids in vivo mit dem Substrat auf Eisenbasis unter Bildung eines Polysaccharid-Eisen-Komplexes mit einer Löslichkeit in einer physiologischen Lösung von nicht weniger als 1 g reagiert.

8. Implantierbare Vorrichtung auf der Basis von resorbierbarem Eisen nach Anspruch 1, wobei das pflanzliche Polysaccharid Ginseng-Polysaccharid, Astragalus-Polysaccharid, Angelika-Polysaccharid, Lycium barbarum-Polysaccharid oder Capsicum-Polysaccharid ist.

9. Implantierbare Vorrichtung auf der Basis von resorbierbarem Eisen nach Anspruch 1, wobei sich das modifizierte hohe Polysaccharid auf einer Oberfläche des Substrats auf Eisenbasis befindet oder das modifizierte hohe Polysaccharid in das Innere des Substrats auf Eisenbasis gefüllt ist.

10. Implantierbare Vorrichtung auf der Basis von resorbierbarem Eisen nach Anspruch 1, wobei die implantierbare Vorrichtung auf der Basis von resorbierbarem Eisen ferner eine abbaubare Polymerschicht umfasst, die auf dem Substrat auf Eisenbasis angeordnet ist, und

die abbaubare Polymerschicht aus mindestens einem Material besteht, das aus einem abbaubaren Polyester und einem abbaubaren Polyanhydrid ausgewählt ist,
wobei der abbaubare Polyester ein beliebiger aus der aus Polymilchsäure, Polyglykolsäure, Poly(milchsäure-co-glykolsäure), Polycaprolacton, Polyhydroxyalkanoaten, Polyacrylaten, Polysuccinaten, Poly(β-hydroxybutyrat) und Polyethylenadipat bestehenden Gruppe ausgewählter ist, oder
der abbaubare Polyester eine physikalische Mischung aus mindestens zweien ist, die aus der aus Polymilchsäure, Polyglykolsäure, Polysuccinat, Poly(β-hydroxybutyrat), Polycaprolacton, Polyethylenadipat, Poly(milchsäure-co-glykolsäure) und Polyhydroxybutyrat-Valerat-Copolymer bestehenden Gruppe ausgewählt sind, oder
der abbaubare Polyester ein beliebiges Copolymer ist, das durch Copolymerisierung von mindestens zweien gebildet ist, die aus Monomeren ausgewählt sind, die Polymilchsäure, Polyglykolsäure, Polysuccinat, Poly(β-hydroxybutyrat), Polycaprolacton, Polyethylenadipat, Poly(milchsäure-co-glykolsäure) und Polyhydroxybutyrat-Valerat-Copolymer bilden, oder
das abbaubare Polyanhydrid mindestens eines ist, das aus der aus Poly(1,3-bis(p-carboxyphenoxy)propan-sebacinsäure), Poly(erucasäure-dimer-sebacinsäure) und Poly(fumarsäure-sebacinsäure) bestehenden Gruppe ausgewählt ist, oder
das abbaubare Polyanhydrid ein beliebiges Copolymer ist, das durch Copolymerisation von mindestens zweien gebildet ist, die aus Monomeren ausgewählt sind, die Poly(1,3-bis(p-carboxyphenoxy)propan-sebacinsäure), Poly(erucasäure-dimer-sebacinsäure) und Poly(fumarsäure-sebacinsäure) bilden,
oder das Material der abbaubaren Polymerschicht ein Copolymer ist, das durch Copolymerisation mindestens zweier Monomere gebildet ist, die den abbaubaren Polyester und das abbaubare Polyanhydrid bilden.

11. Implantierbare Vorrichtung auf der Basis von resorbierbarem Eisen nach Anspruch 10, wobei die abbaubare Polymerschicht ferner einen Arzneimittelwirkstoff umfasst, der mindestens einer ist, der aus der aus antiangiogenen Arzneimitteln, Thrombozytenaggregationshemmern, antithrombotischen Arzneimitteln, entzündungshemmenden Arzneimitteln und Arzneimitteln zur Antisensibilisierung bestehenden Gruppe ausgewählt ist.

12. Implantierbare Vorrichtung auf der Basis von resorbierbarem Eisen nach Anspruch 11, wobei

das antiangiogene Arzneimittel aus Paclitaxel und/oder Rapamycin ausgewählt ist,
der Thrombozytenaggregationshemmer Cilostazol ist,
das antithrombotische Arzneimittel Heparin ist,
das entzündungshemmende Arzneimittel Dexamethason ist und
das Arzneimittel zur Antisensibilisierung mindestens eines ist, das aus Calciumgluconat, Chlorphenamin und Cortison ausgewählt ist.

**Revendications**

1. Dispositif implantable absorbable à base de fer, comprenant :

un substrat à base de fer, et
un polysaccharide élevé modifié attaché au substrat à base de fer,
le polysaccharide élevé étant au moins l'un choisi parmi le glucane, le chitosane, les polysaccharides végétaux, l'amidon, la dextrine, la cellulose, le glycogène, la chitine, la tunicine, l'inuline, l'agar, la gomme arabique, l'acide hyaluronique, la gomme géllane, le curdlane, la gomme de xanthane, la pectine, le glucomannane de konjac, la gomme arabique, la lichénine, l'alginate, le polysaccharide spirulina, le sulfate de chondroïtine, le sulfate de dermatane, le sulfate de kératane, l'héparine et le sulfate d'héparane, et
le polysaccharide élevé modifié étant un polysaccharide élevé modifié de manière hydrophobe.

2. Dispositif implantable absorbable à base de fer selon la revendication 1, le polysaccharide élevé modifié étant formé par greffage d'un groupe hydrophobe à un polysaccharide élevé.

3. Dispositif implantable absorbable à base de fer selon la revendication 2, le groupe hydrophobe étant un groupe hydrocarbyle comprenant 8 à 24 atomes de carbone ; ou

le groupe hydrophobe étant un groupe hydrocarbyle contenant de l'aryle et/ou de l'hydroxyle et/ou du carboxyle et/ou un groupe ester et/ou un groupe isocyanate et/ou un groupe éther et/ou un groupe amine et/ou de l'acyle, ou un groupe hydrocarbyle contenant une double liaison ; ou
le groupe hydrophobe étant au moins l'un choisi parmi le polyoxypropényle, un perfluoroalkyle à longue chaîne et un groupe siloxane.

4. Dispositif implantable absorbable à base de fer selon la revendication 2, le groupe hydrophobe réagissant avec de l'hydroxyle, du carboxyle ou le groupe amino du polysaccharide élevé pour se greffer au polysaccharide élevé.

5. Dispositif implantable absorbable à base de fer selon la revendication 1, la solubilité du polysaccharide élevé modifié dans de l'eau à 37°C étant inférieure ou égale à 0,01 g, et la solubilité du polysaccaride élevé modifié dans un solvant organique étant supérieure ou égale à 0,1 g.

6. Dispositif implantable absorbable à base de fer selon la revendication 1, le temps de dégradation du polysaccharide élevé modifié in vivo étant supérieur à 3 mois, et
la détermination du temps de dégradation incluant : l'immersion du dispositif implantable dans une solution saline tamponnée au phosphate dans un volume qui est 5 fois le volume du dispositif implantable, et l'agencement du dispositif implantable dans un environnement de bain d'eau thermostatique à 37°C agité à 60 tours par minute pour plus de 3 mois.

7. Dispositif implantable absorbable à base de fer selon la revendication 1, un produit de dégradation du polysaccharide élevé modifié in vivo réagissant avec le substrat à base de fer de manière à former un complexe de polysaccharide-fer présentant une solubilité dans une solution physiologique non inférieure à 1 g.

8. Dispositif implantable absorbable à base de fer selon la revendication 1, le polysaccharide végétal étant du polysaccharide de ginseng, du polysaccharide d'astragale, du polysaccharide d'angélique, du polysaccharide de lycium barbarum ou du polysaccharide de capsicum.

9. Dispositif implantable absorbable à base de fer selon la revendication 1, le polysaccharide élevé modifié se trouvant sur une surface du substrat à base de fer, ou le polysaccharide élevé modifié étant rempli à l'intérieur du substrat à base de fer.

**10.** Dispositif implantable absorbable à base de fer selon la revendication 1, le dispositif implantable absorbable à base de fer comprenant en outre une couche de polymère dégradable agencée sur le substrat à base de fer, et

la couche de polymère dégradable étant réalisée en au moins un matériau choisi parmi un polyester dégradable et un polyanhydride dégradable,
le polyester dégradable étant l'un choisi dans le groupe constitué d'acide polylactique, d'acide polyglycolique, de poly(acide lactique-co-glycolique), de polycaprolactone, de polyhydroxyalcanoates, de polyacrylates, de polysuccinates, de poly(β-hydroxybutyrate) et d'adipate de polyéthylène ; ou
le polyester dégradable étant un mélange physique d'au moins deux substances choisies dans le groupe constitué d'acide polylactique, d'acide polyglycolique, de polysuccinate, de poly(β-hydroxybutyrate), de polycaprolactone, d'adipate de polyéthylène, de poly(acide lactique-co-glycolique) et d'un copolymère de polyhydroxybutyrate-valérate ; ou
le polyester dégradable étant un copolymère quelconque formé par copolymérisation d'au moins deux monomères choisis parmi ceux formant de l'acide polylactique, de l'acide polyglycolique, du polysuccinate, du poly(β-hydroxybutyrate), de la polycaprolactone, de l'adipate de polyéthylène , du poly(acide lactique-co-glycolique) et le copolymère de polyhydroxybutyrate-valérate ; ou
le polyanhydride dégradable étant au moins l'un choisi dans le groupe constitué de poly(1,3-bis(p-carboxyphénoxy) propane-acide sébacique), de poly(dimère d'acide érucique-acide sébacique) et de poly(acide fumarique-acide sébacique) ; ou
le polyanhydride dégradable étant un copolymère quelconque formé par copolymérisation d'au moins deux monomères choisis parmi ceux formant du poly(1,3-bis(p-carboxyphénoxy) propane-acide sebacique), du poly(dimère d'acide érucique-acide sébacique) et du poly(acide fumarique-acide sébacique) ;
ou le matériau de la couche de polymère dégradable étant un copolymère formé par copolymérisation d'au moins deux monomères formant le polyester dégradable et le polyanhydride dégradable.

**11.** Dispositif implantable absorbable à base de fer selon la revendication 10, la couche polymère dégradable comprenant en outre un médicament actif qui est au moins l'un choisi dans le groupe constitué de médicaments anti-angiogéniques, de médicaments anti-plaquettaires, de médicaments anti-thrombotiques, de médicaments anti-inflammatoires et de médicaments anti-sensibilisants.

**12.** Dispositif implantable absorbable à base de fer selon la revendication 11,

le médicament anti-angiogénique étant choisi parmi le paclitaxel et/ou la rapamycine ;
le médicament anti-plaquettaire étant le cilostazol ;
le médicament anti-thrombotique étant l'héparine ;
le médicament anti-inflammatoire étant le dexaméthasone ; et
le médicament anti-sensibilisant étant au moins l'un choisi parmi le gluconate de calcium, la chlorphénamine et la cortisone.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2009202610 A1
- US 2004037886 A1
- EP 3064232 A